# EUROPÄISCHE PATENTSCHRIFT

(11) **EP 1 731 086 B1**
(45) Veröffentlichungstag und Bekanntmachung des Hinweises auf die Patenterteilung: **27.11.2013**
(21) Anmeldenummer: 06009028.9
(22) Anmeldetag: 02.05.2006
(51) Int. Cl.: A61B 3/135, A61B 3/10

(54) **Verfahren zum Betrieb eines ophthalmologischen Analysesystems**
Method of operating an ophthalmological analysis system
Méthode d'opération d'un système d'analyse ophtalmologigique

(30) Priorität: 07.06.2005 DE 102005026371
(43) Veröffentlichungstag der Anmeldung: 13.12.2006
(73) Patentinhaber: Oculus Optikgeräte GmbH, 35582 Wetzlar-Dutenhofen (DE)
(72) Erfinder: Köst, Gert, 30171 Hannover (DE)
(74) Vertreter: von den Steinen, Axel

(56) Entgegenhaltungen:
- EP-A1- 1 074 214
- EP-A2- 1 275 337
- WO-A-02/45578
- DE-A1- 4 321 796
- DE-A1- 10 307 741
- GB-A- 2 112 171
- US-A- 4 632 528
- US-A- 5 886 768
- US-A1- 2002 101 567
- US-A1- 2005 122 474
- B. DICK, J. NOVAK, A. GILG, M. MAUS, T. NEUMANN, S. NAROO, J. HOLLADAY: "Interpretation of Scheimpflug based anterior segment imaging and mapping" EUROTIMES, [Online] 1. Februar 2005 (2005-02-01), XP002412373 Eye to Eye Gefunden im Internet: URL:www.oculus.de/en/downloads/dyn/sonstig e/sonstige/2_eye_eye_supplement-pdf> [gefunden am 2006-12-18]
- "Pentacam Die neue Sicht ins Auge" AUGENLICHT VISIONCARE, [Online] 2005, XP002412374 Gefunden im Internet: URL:www.oculus.de/de/downloads/dyn/prod_so nstige/penta/augenlicht_2_2005.pdf> [gefunden am 2006-12-19]
- DRAGOMIRESCU V ET AL: "DEVELOPMENT OF A NEW EQUIPMENT FOR ROTATING SLIT IMAGE PHOTOGRAPHY ACCORDING TO SCHEIMPFLUG'S PRINCIPLE" Januar 1978 (1978-01), INTERDISCIPLINARY TOPICS IN GERONTOLOGY, KARGER, BASEL, CH, PAGE(S) 118-130 , XP000613975 ISSN: 0074-1132 * Seite 1 - Seite 2 *

## Beschreibung

Die Erfindung betrifft ein Verfahren zum Betrieb eines ophthalmologischen Analysesystems.

Geräte zur Augenuntersuchung mit Scheimpflugkameras sind aus dem Stand der Technik bekannt und werden üblicherweise zur Diagnose der vorderen Augenkammer eingesetzt. Diese Geräte ermöglichen eine besondere Form der Spaltlampenuntersuchung, wobei die beleuchtete Ebene des Auges mit der Scheimpflugaufnahmeeinrichtung aufgenommen wird. Diese Geräte zur Augenuntersuchung weisen neben der Scheimpflugaufnahmeeinrichtung auch noch eine Spaltprojektionseinrichtung zur Spaltbeleuchtung des Auges auf. Derartige Spaltprojektionseinrichtungen sind ebenfalls aus dem Stand der Technik bekannt. Das Prinzip des Spaltprojektors beruht darauf, dass die brechenden Medien im Auge nicht glasklar sind, sondern an ihnen, insbesondere im kurzwelligen Anteil des sichtbaren Lichts, eine deutliche Streuung erfolgt. Dies führt dazu, dass ein scharf gebündelter Lichtstrahl, d.h. der projizierte Lichtspalt, den man durch die optischen Medien des Auges schickt, bei seitlicher Betrachtung sichtbar wird. Dieser Effekt ist ähnlich der Lichtbrechung beim Durchtritt von Scheinwerferlicht durch Nebel. Da die verschiedenen Bestandteile des Auges unterschiedlich starke Lichtstreuungen aufweisen, können durch diese Untersuchungen Aussagen über den Aufbau des Auges gemacht werden. Zur Spaltbeleuchtung wird ein spaltförmiges Lichtbündel hoher Lichthelligkeit und möglichst hoher Farbtemperatur verwendet.

Die mittels der Spaltprojektionseinrichtung erleuchtete Ebene im Auge wird mittels der Scheimpflugaufnahmeeinrichtung aufgenommen. Eine solche Scheimpflugaufnahmeeinrichtung ist eine Aufnahmeeinrichtung, die der Scheimpflugbedingung genügt. Die Scheimpflugbedingung fordert, dass die Projektionsebene, dies ist hier die mit der Spaltprojektionseinrichtung erleuchtete Ebene im Auge, die Hauptebene des Linsensystems und die Bildebene zwischen einer gemeinsamen Achse schneiden. Durch das Kippen der Bildebene gegenüber der Hauptebene des Linsensystems, kann die Projektionsebene in einer beliebigen räumlichen Lage sein, wobei in der Schärfentiefenzone Bildpunkte erfasst werden können, die bei senkrechter Projektionsebene nicht zugleich scharf abgebildet werden können.

Ein Verfahren beruht auf dem Grundgedanken eine Spaltprojektionseinrichtung zur Spaltbeleuchtung des Auges und eine Scheimpflugaufnahmeeinrichtung zur Aufnahme von Schnittbildern des Auges zu verwenden, die gemeinsam um eine Achse rotierend verschwenkt werden können. Die Schwenkachse von Scheimpflugaufnahmeeinrichtung und Spaltprojektionseinrichtung fällt dabei im Wesentlichen mit der optischen Achse des Auges zusammen. Auf diese Weise können im Rahmen einer Messreihe Scheimpflugaufnahmen in verschiedenen Ebenen des Auges gemacht werden, so dass diese jeweils zweidimensionalen Scheimpflugaufnahmen gemeinsam dreidimensional Informationen über den Aufbau des Auges enthalten.

Üblicherweise werden die Scheimpflugaufnahmen in digitalisierter Form aufgenommen. D.h. jede Scheimpflugaufnahme einer Schnittebene durch das Auge wird als einzelner Bilddatensatz gespeichert. Diese digitalisierte Aufnahmetechnik ermöglicht eine digitale Bilddatenanalyse, so dass aus den verschiedenen Bilddatensätzen in einer Bilddatenanalyseeinrichtung der Aufbau des Auges bzw. der Aufbau von Bestandteilen des Auges abgeleitet werden kann. Das dabei erhaltene mehrdimensionale Beschreibungsmodel des untersuchten Bestandteil des Auges wird als Ergebnisdatensatz abgespeichert. Diese Ergebnisdaten können anschließend dann in geeigneter Form, beispielsweise als dreidimensionales Geometriemodel an einer Datenausgabeeinrichtung, beispielsweise einem Bildschirm, angezeigt werden.

Welche Art von Untersuchungen am menschlichen Auge mit solchen Verfahren durchgeführt werden, insbesondere welche Bestandteile des Auges untersucht werden, ist grundsätzlich beliebig. Nach einer Variante wird das Verfahren eingesetzt, um die Achslänge des Auges zwischen Hornhaut und Netzhaut abzuleiten. Als Achslänge des Auges soll dabei der Abstand verstanden werden, der zwischen dem Eintrittspunkt eines Lichtstrahls am Apex der Hornhaut bis zum Auftreffpunkt an der Netzhaut liegt. Der Achslängenwert ist insbesondere für die Berechnung von künstlichen Intraocularlinsen, die operativ ins Auge eingesetzt werden, von Bedeutung, den die Brechkraft der Intraocularlinsen muss so gewählt werden, dass auf der Netzhaut ein scharfes Bild entsteht. Da alle anderen Werte zur Berechnung einer Intraocularlinse (Vorderkammertiefe, Hornhautbrechkraft, etc.) ebenfalls unter Einsatz des ophthalmologischen Analysesystems mit rotierbarer Scheimpflugaufnahmeeinrichtung bestimmt werden können, ist die Ableitung der Achslänge des Auges mit dem erfindungsgemäßen Verfahren von besonderem Vorteil, da dieser Wert bisher nur mit sehr umständlichen Messverfahren (Ultraschallmessung, Interferenzmessung) in separaten Untersuchungsgeräten bestimmt werden kann. Durch die Bestimmung der Achslänge des Auges mit dem erfindungsgemäßen Verfahren sind im Ergebnis alle zur Berechnung einer Intraocularlinse benötigten Messwerte mit einem einzigen Untersuchungsgerät bestimmt, was eine erhebliche Erleichterung darstellt.

Zur Bestimmung der Achslänge des Auges können entsprechend geeignete Untersuchungsanordnungen, beispielsweise zur Interferenzmessung oder zur Ultraschallmessung, in das zur Schnittbilduntersuchung vorgesehene ophthalmologischen Analysesystem integriert werden. Alternativ dazu ist die Vermessung der Achslänge des Auges auch durch Auswertung der die Schnittbilder enthaltenen Datensätze möglich. Um die Achslänge des Auges möglichst einfach aus den die Schnittbilder enthaltenen Datensätzen bestimmen zu können, kann das Auge über die volle Tiefe von der Hornhaut bis zur Netzhaut im Tiefenschärfenbereich der Scheimpflugaufnahmeeinrichtung abgebildet werden. Dies kann insbesondere dadurch erreicht werden, dass die Scheimpflugaufnahmeeinrichtung mit sehr flachem Aufnahmewinkel gegenüber der Hauptachse des Auges eingestellt wird.

Nach einer Variante des Untersuchungsverfahrens werden aus den Bilddatensätzen die Dicke der natürlichen Augenlinse des Auges oder die Position einer die natürliche Augenlinse ersetzenden, künstlichen Intraocularlinse im Auge abgeleitet. Diese Art der Untersuchung ist für das Akkomodationsvermögen des Auges von großer Bedeutung, da die natürliche Augenlinse in ihrer Dicke verformbar ist, wodurch sich das Auge an unterschiedliche Gegenstandsweiten anpassen kann. Diese Akkomodation wird bei Ersatz der natürlichen Augenlinse durch eine künstliche Intraocularlinse durch geeignete Verstellung der Intraocularlinse relativ zur Netzhaut realisiert.

Um das Akkomodationsverhalten der natürlichen Augenlinse bzw. der künstlichen Intraocularlinse untersuchen zu können, ist es besonders vorteilhaft, wenn der zur Verformung der natürlichen Augenlinse bzw. zur Verstellung der die natürliche Augenlinse ersetzenden Intraocularlinse vorgesehene Muskel während einer Messreihe in unterschiedliche Erregungszustände versetzt wird. Auf diese Weise kann die Verformung der Augenlinse bzw. die Verstellung der Intraocularlinse in Abhängigkeit der Erregung des Akkomodationsmuskels im Auge durch die erfindungsgemäße Bilddatenanalyse bestimmt werden.

Um während der Durchführung des Untersuchungsverfahrens die gewünschte Erregung des Akkomodationsmuskels mit unterschiedlichen Erregungszuständen realisieren zu können, kann die im Analysesystem vorgesehene Fixationsmarke eingesetzt werden. Diese Fixationsmarke, beispielsweise ein Lichtpunkt, wird vom Patienten während der Untersuchung mit dem zu untersuchenden Auge anvisiert, um das Auge auf diese Weise in einer definierten Position zu fixieren. Da das Auge dabei unwillkürlich die scharfe Abbildung der Fixationsmarke auf die Netzhaut durch Akkomodation der natürlichen Augenlinse bzw. Verstellung der künstlichen Intraocularlinse versucht, kann durch Änderung des tatsächlichen oder virtuellen Abstands zwischen Auge und Fixationsmarke der Akkomodationsmuskel in der gewünschten Weise variabel erregt werden. Wird die Fixationsmarke beispielsweise in einem Nahpunkt, d.h. in einem im Abstand von ungefähr 30 cm, vor dem Auge angeordnet, wird die Augenlinse bzw. die Intraocularlinse durch dem Akkomodationsmuskel derart verformt bzw. verstellt, dass die Fixationsmarke scharf auf die Netzhaut abgebildet wird. Wird die Fixationsmarke anschließend in einem Fernpunkt, beispielsweise in einem virtuellen Abstand von 5 m, vor dem Auge angeordnet, ändert sich die Muskelspannung des Akkomodationsmuskels entsprechend, so dass durch die geänderte Dicke der Augenlinse bzw. durch die geänderte Position der Interokularlinse die Fixationsmarke wiederum scharf auf die Netzhaut abgebildet wird. Im Ergebnis hat man also durch die Veränderung des Abstands zwischen Auge und Fixationsmarke die Möglichkeit den Akkomodationsmuskel in der gewünschten Weise zu erregen.

Die Änderung des tatsächlichen Abstands zwischen Auge und Fixationsmarke erfordert einen vielfach nur schwer zu realisierenden Geräteaufbau, da ein Gerät mit einer Achslänge von beispielsweise mehreren Metern außerordentlich unhandlich ist. Es ist deshalb vorzuziehen, den Abstand zwischen Auge und Fixationsmarke nur virtuell durch Anordnung von zumindest einer Linse im Strahlengang zwischen Fixationsmarke und Auge zu variieren. Dies bedeutet mit anderen Worten, dass der tatsächliche Abstand zwischen Auge und Fixationsmarke unverändert bleibt. Durch die Anordnung einer Linse kann die Fixationsmarke jedoch auf eine virtuelle Bildebene projiziert werden, so dass die Augenlinse bzw. die Intraocularlinse nicht mehr auf die tatsächliche Fixationsmarke sondern auf die virtuelle Abbildung der Fixationsmarke in die virtuelle Bildebene scharf gestellt wird. Auf diese Weise können auch große virtuelle Abstände zwischen Auge und Fixationsmarke simuliert werden, ohne dass der tatsächliche Abstand zwischen Auge und Fixationsmarke entsprechend groß sein muss.

Nach einer Variante des Untersuchungsverfahrens kann durch Analyse der Bilddatensätze auch das Vorhandensein eines Keratoconus an der Hornhaut abgeleitet werden. Ein solcher Keratoconus stellt eine krankheitsbedingte Verformung der Hornhaut dar, die Störungen des optischen Systems im Auge hervorruft. Verursacht wird diese Verformung durch Ausdünnungen in der Hornhaut, so dass sich die Hornhaut dadurch aufgrund des Innendrucks im Auges üblicherweise konusartig verformt.

Um das Vorhandensein eines Keratoconus ableiten zu können ist es nach einer ersten Variante vorgesehen, dass der dreidimensionale Verlauf der Hornhautdicke aus den Bilddatensätzen abgeleitet wird. Werden in diesem dreidimensionalen Verlauf der Hornhautdicke Schwankungen oberhalb einer bestimmten Toleranzgrenze nachgewiesen, so kann vom Vorhandensein eines Keratoconus ausgegangen werden.

Alternativ bzw. additiv zur Bestimmung des Verlaufs der Hornhautdicke kann auch die dreidimensionale Geometrie der Hornhautvorderfläche aus den Bilddatensätzen abgeleitet werden. Zeigt die Hornhautvorderfläche eine im Wesentlichen konusartige Geometrie, so ist wiederum vom Vorhandensein eines Keratoconus auszugehen.

Bei der Keratoconuserkennung ist es dabei besonders vorteilhaft, wenn im Analysesystem Vergleichswerte vorgegeben sind, die beispielsweise den gesunden Zustand der Hornhaut bzw. den pathologischen Zustand der Hornhaut bei Keratoconus beschreiben. Die nach Durchführung des Untersuchungsverfahrens erhaltenen Ist-Werte des Verlaufs der Hornhautdicke und/oder der Geometrie der Hornhautvorderfläche können dann mit diesen hinterlegten Vergleichswerten verglichen werden, um anhand des Vergleichsergebnisses auf das Vorhandensein eines Keratoconus schließen zu können. Diese Art von Untersuchung ist von großer Bedeutung, da der Keratoconus eine Kontraindikation für viele Behandlungen, insbesondere die Korrektur von Fehlsichtigkeiten mittels eines Eximerlasers, darstellt.

Nach einer Variante wird aus den Bilddatensätzen die Geometrie der Augenvorkammer zwischen Hornhaut und Iris abgeleitet. Diese Art der Untersuchung ist insbesondere dann von Bedeutung, wenn in die Augenvorkammer eine phake Intraocularlinse in Ergänzung der verbleibenden natürlichen Augenlinse implantiert werden soll. Durch die Bestimmung der Geometrie der Augenvorkammer ist es nämlich möglich, dass vor Durchführung der Implantationsoperation eine Simulation durchgeführt wird, mit der die Anordnung einer phaken Intraocularlinse in der Augenvorkammer simuliert werden kann. Nur wenn sich bei der Simulation ergibt, dass in der Augenvorkammer ausreichend Platz zur Aufnahme der geplante Intraocularlinse vorhanden ist, wird die Operation zur Implantierung der Intraocularlinse durchgeführt.

Nach einer Variante werden aus den Bilddatensätzen die Geometrie der Iris und des unmittelbar daran anschließenden Teils des Glaskörpers abgeleitet. Diese Art der Untersuchung ermöglicht es die Anordnung einer künstlichen Intraocularlinse im Bereich hinter der Iris im Glaskörper zu simulieren.

Nach einer Variante kann das erfindungsgemäße Untersuchungsverfahren auch zur Bestimmung der dreidimensionalen Geometrie von Trübungen der Augenlinse (Katarakt) eingesetzt werden. Die natürliche Augenlinse wird mit zunehmenden Alter nämlich immer trüber, wobei der Grad der Eintrübung und die Geometrie der Eintrübung individuell verschieden ist. Diese als Katarakt bezeichneten Eintrübungen stören das optische System des Auges, da sie zu einer verminderten Sehschärfe und einer verminderten Kontrastempfindlichkeit, sowie zu einer erhöhten Blendempfindlichkeit führen. Wird eine zu starke Trübung der Augenlinse festgestellt, so kann diese als Katarakt bezeichnete Erkrankung durch Entfernen der natürlichen Augenlinse und Implantierung einer künstlichen Intraocularlinse behandelt werden.

Besonders vorteilhaft ist es, wenn die bei der Katarakterkennung eigenen Ist-Werte der Trübungsgeometrie mit im Analysesystem vorgegebenen Vergleichswerten verglichen werden, um durch Auswertung des Vergleichsergebnisses die Art des Katarakts klassifizieren zu können.

EP1074214 von Oculus beschreibt die sogenannte Pentacam, bei der eine Spaltprojektionseinrichtung und eine Scheimpflugaufnahmeeinrichtung gemeinsam um die optische Achse des Auges rotieren um Schnittbilder des Auges aufzunehmen. Verfahren, um dieses Gerät zu betreiben werden beschrieben in einer Veröffentlichung von Burkhard Dick (Eurotimes, Eye to Eye, Februar 2005). Weitere Geräte dieser Art werden beschrieben in DE4321796 von Nidek, sowie UK2112171 von Carl Zeiss.

Ausgehend von diesem Stand der Technik ist es Aufgabe der vorliegenden Erfindung, ein neues Verfahren zum Betrieb solcher Analysesysteme vorzuschlagen, mit dem bei einfacher Handhabung ein Brechungsverhältnis einer Augenlinse abgeleitet werden kann.

Das erfindungsgemäße Verfahren beruht auf dem Grundgedanken, während einer Messreihe im Bereich zwischen Spaltprojektionseinrichtung und Auge eine Rasterblende anzuordnen, so dass der Beleuchtungsspalt durch die Rasterblende in mehrere Spaltabschnitte aufgeteilt wird. Das Licht der Spaltprojektionseinrichtung bildet auf diese Weise ein Streulichtmuster dessen einzelne Strahlen entlang getrennter Strahlengänge durch das Auge verlaufen. Durch Aufnahme und Bilddatenauswertung des Lichtstrahlenverlaufs kann das Brechungsverhältnis der Augenlinse, insbesondere der Brechungsindex des die Augenlinse bildenden Gewebes, abgeleitet werden. Außerdem ist es dadurch, dass das Auge während der Messreihe mit Streulichtmuster auf einen Fixationspunkt in unendlicher Entfernung fixiert wird, möglich, dass aus den das Streulichtmuster enthaltenen Bilddatensätzen die Achslänge des Auges zwischen Hornhaut und Netzhaut abgeleitet wird.

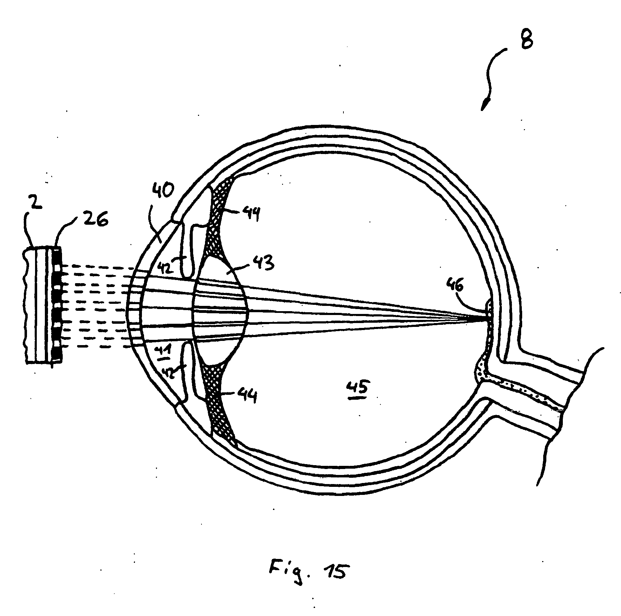

Das erfindungsgemäße Verfahren wird nachfolgend anhand der Zeichnung Fig 15 beispielhaft erläutert.

Es zeigen:
- **Fig. 1**: ein Gerät zur Durchführung des erfindungsgemäßen Verfahrens in Ansicht von vorne;
- **Fig. 2**: das Gerät gemäß **Fig. 1** im Querschnitt entlang der Schnittlinie II - II;
- **Fig. 3**: das Gerät gemäß **Fig. 1** in Ansicht von oben;
- **Fig. 4**: die Spaltprojektionseinrichtung des Geräts gemäß **Fig. 1** im Querschnitt;
- **Fig. 5**: Einzelheiten eines Schleifringübertragers im Gerät gemäß **Fig. 1****;**
- **Fig. 6**: den Aufbau eines mit dem Gerät gemäß **Fig. 1** zu untersuchenden Auges im schematisierten Querschnitt;
- **Fig. 7**: die Funktionsweise des Akkomodationsmuskels bei Verformung der Augenlinse im schematisierten Querschnitt;
- **Fig. 8**: die Funktionsweise des Akkomodationsmuskels bei Verstellung einer künstlichen, akkomodativen Intraocularlinse im schematisierten Querschnitt;
- **Fig. 9**: den Dickenverlauf einer gesunden Hornhaut im schematisierten Querschnitt;
- **Fig. 10**: den Dickenverlauf einer Hornhaut mit Keratoconuserkrankung im schematisierten Querschnitt;
- **Fig. 11**: die Anordnung einer phaken Intraocularlinse in der Augenvorkammer eines Auges im schematisierten Querschnitt;
- **Fig. 12**: die Anordnung einer künstlichen Intraocularlinse zum Ersatz der Augenlinse im Bereich unmittelbar hinter der Iris im schematisierten Querschnitt;
- **Fig. 13**: ein Auge mit ungetrübter Augenlinse im schematisierten Querschnitt;
- **Fig. 14**: ein Auge mit getrübter Augenlinse (Katarakterkrankung) im schematisierten Querschnitt;
- **Fig. 15**: ein Auge bei Projektion eines Streulichtmusters mit einer Rasterblende im schematisierten Querschnitt.

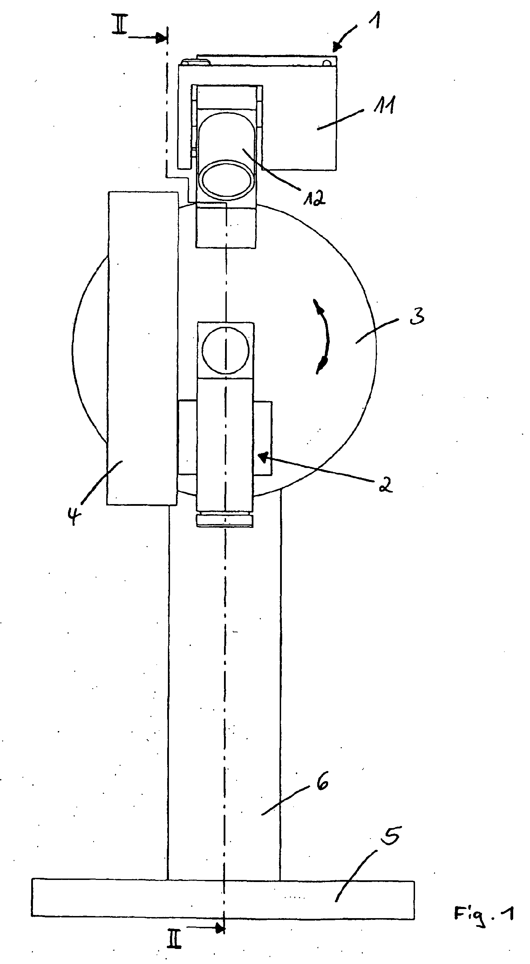

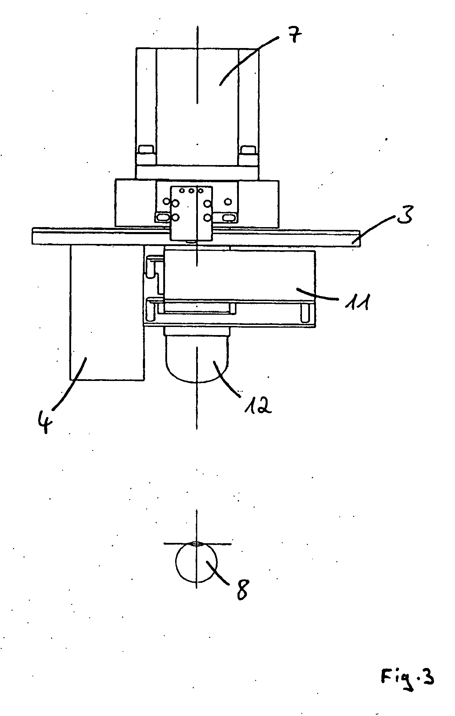

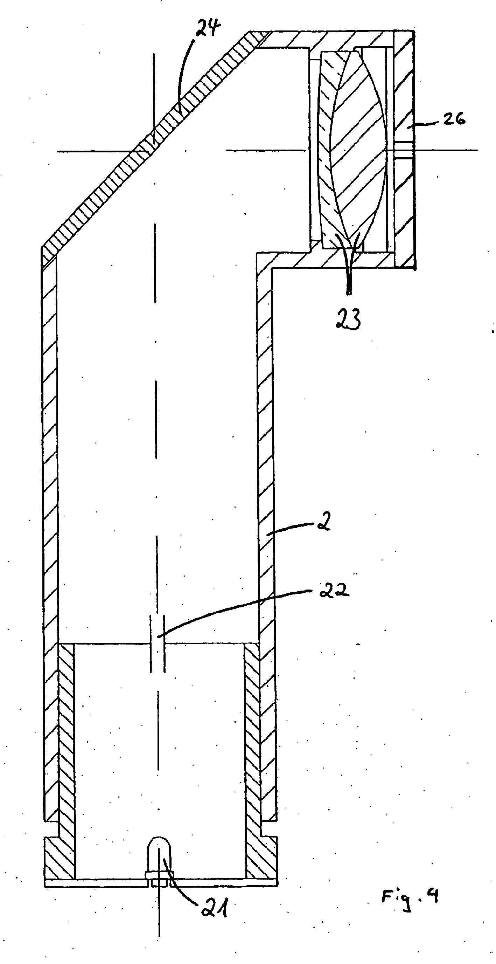

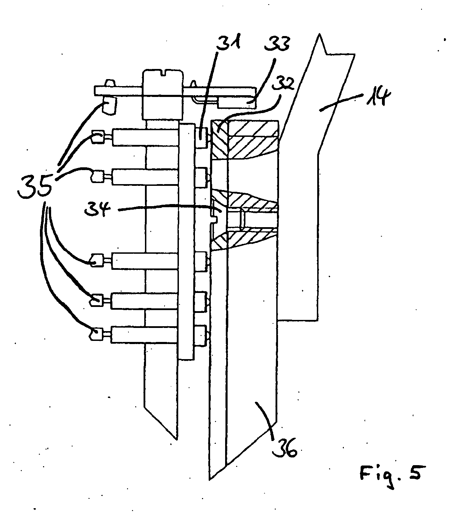

**Fig. 1** bis **Fig. 5** zeigen ein Gerät mit einer Scheimpflugaufnahmeeinrichtung 1 und einer Spaltprojektionseinrichtung 2, die gemeinsam an einem drehbar gelagerten Rotor 3 befestigt sind. Dieses Gerät kann eingesetzt werden, um das erfindungsgemäße Verfahren durchzuführen. Selbstverständlich sind dazu aber auch anders ausgebildete Geräte mit rotierender Scheimpflugaufnahmeeinrichtung geeignet. Soweit die Achslänge des Auges nicht aus den Bilddaten der mit der Scheimpflugaufnahmeeinrichtung 1 aufgenommenen Schnittbilder abgeleitet werden, kann dazu auch ein anderes, entsprechend geeignetes Untersuchungsgerät, beispielsweise ein Ultraschallmessgerät oder ein Interferenzmessgerät, zusätzlich in das Gerät mit der Scheimpflugaufnahmeeinrichtung 1 der Spaltprojektionseinrichtung integriert werden.

Der Rotor 3 ist an einem Ständer 6 gelagert und kann mit einem Motor 7 rotatorisch angetrieben werden. Bei dem Motor 7 handelt es sich vorzugsweise um einen Schrittmotor. Die Befestigung von Spaltprojektionseinrichtung 2 und Scheimpflugaufnahmeeinrichtung 1 am Rotor 3 ist mittels Halter 14 und 25 realisiert. Die optische Achse der aus der Spaltprojektionseinrichtung 2 austretenden Strahlen fällt mit der Drehachse des Rotors 3 zusammen. Das zu untersuchende Auge 8 ist während der Untersuchung dabei so anzuordnen, dass die Hauptachse des Auges mit der optischen Achse des Spaltprojektors 2 und der Drehachse des Rotors 3 zusammenfällt.

Der Spaltprojektor 2 weist als Lichtquelle eine Diodenreihe 21 auf. Diese Diodenreihe 21 beleuchtet eine Spaltblende 22, wodurch die Spaltblende gemäß dem köhlerschen Abbildungsschema über einem Spiegel 24 in dem Projektor-Linsensystem 23 abgebildet wird. Im Strahlengang des Lichtspalts kann dabei zusätzlich eine Rasterblende 26 angeordnet werden, um auf diese Weise den Lichtspalt in mehrere Bildspaltabschnitte aufzuteilen, so dass ein Streulichtmuster auf das Auge projiziert wird.

Das in dem Projektor-Linsensystem 23 gebündelte Licht des Lichtspalts durchläuft das Auge 8 in einer Ebene entlang getrennter Strahlenbündel. Diese mit dem Lichtspalt beleuchtete Ebene kann mittels der Scheimpflugaufnahmeeinrichtung 1 aufgenommen werden.

Dazu weist die Scheimpflugaufnahmeeinrichtung 1 ein Kamera-Linsensystem 12 auf, welches vor dem Gehäuse 11 montiert ist. Im Gehäuse 11 der Scheimpflugaufnahmeeinrichtung 1 ist in einer Bildebene 13 ein CCD-Chip angeordnet, der digitalisierte Schnittbilder des Auges aufnimmt. Um der Scheimpflugbedingung genüge zu tun, sind die Bildebene 13, die Hauptebene des Kamera-Linsensystems 12 und die beleuchtete Projektionsebene im Auge 8 des Probanten so zueinander geneigt, dass sie sich in einer gemeinsamen Achse 9 schneiden.

Das mit der Scheimpflugaufnahmeeinrichtung 1 aufgenommene digitalisierte Schnittbild wird nach der Aufnahme als Bilddatensatz gespeichert und kann in einer geeigneten Bilddatenanalyseeinrichtung, die beispielsweise auf einem Industrie-PC als Software installiert ist, ausgewertet werden. Nach Aufnahme eines Schnittbildes wird der Rotor 3 jeweils einstückweit um seine Rotationsachse verschwenkt und dann in einer weiteren Schnittebene ein weiteres digitales Schnittbild des Auges 8 aufgenommen.

Zur Signalübertragung sind zwei Schleifringe 32 vorgesehen, die am Rotor 3 mittels Schrauben 34 befestigt sind. Am Ständer 6 sind weitere Schleifringkontakte 31 befestigt, die ebenfalls der Signalübertragung dienen. Über die Anschlussleitung 35 können die Signale zu der nicht dargestellten Bilddatenanalyseeinrichtung übertragen werden.

Zusätzlich zu den beiden Schleifringübertragern für die Signalübertragung sind weitere drei Schleifringe 32 bzw. weitere drei Schleifkontakte 31 an dem Rotor 3 bzw. an dem Ständer 6 vorgesehen, die der Spannungsversorgung dienen. Außerdem ist am Ständer 6 ein Sensor 33 vorgesehen, mit dem der Drehwinkel des Rotors 3 gemessen werden kann. Somit kann bei jeder Schnittbildaufnahme die Winkelstellung der Scheimpflugaufnahmeeinrichtung 1 relativ zu einer Referenzposition bestimmt werden.

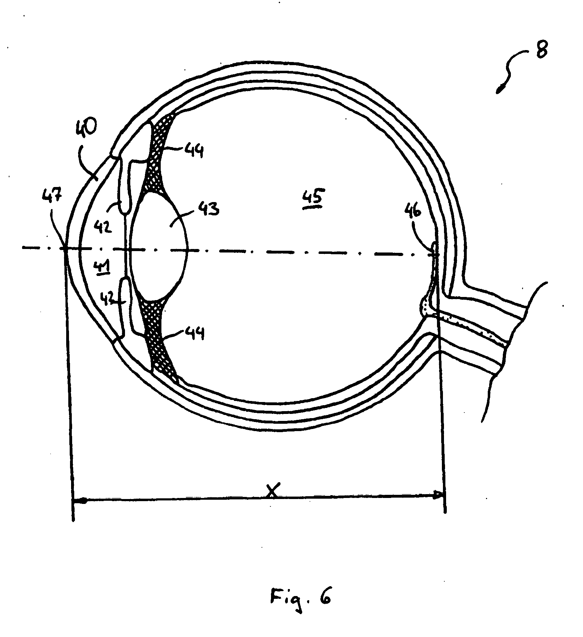

In **Fig. 6** ist der Aufbau des zu untersuchenden Auges 8 schematisiert dargestellt. Hinter der Hornhaut 40 befindet sich die Augenvorkammer 41, die nach hinten durch die Iris 42 begrenzt ist. Unmittelbar hinter der Iris 41 befindet sich die natürliche Augenlinse 43, deren Form und insbesondere Dicke durch Anspannung des Akkomodationsmuskels 44 verändert werden kann. Nach Durchlaufen des Glaskörpers 45 werden die in das Auge 8 fallenden Lichtstrahlen auf die Netzhaut 46 fokussiert. Durch das erfindungsgemäße Untersuchungsverfahren kann die Achslänge X zwischen dem Apex 47 der Hornhaut 40 und der Netzhaut 46 bestimmt werden. Dazu werden in mehreren Rotationswinkeln des Rotors 3 mehrere digitalisierte Schnittbilder des Auges 8 angefertigt, wobei zwischen der Spaltprojektionseinrichtung 2 und der Scheimpflugaufnahmeeinrichtung 1 ein möglichst flacher Bildwinkel α eingestellt wird. Der

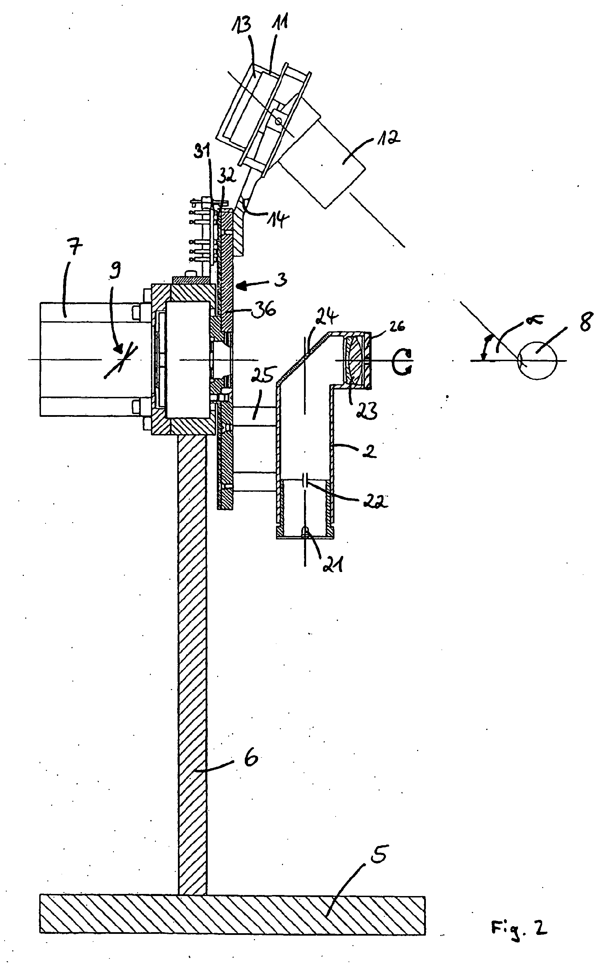

Bildwinkel α sollte dabei möglichst erheblich kleiner als in Fig. 2 sein.

Insbesondere Bildwinkel α im Bereich von 5° bis 10° sind besonders zur

Bestimmung der Achse X geeignet.

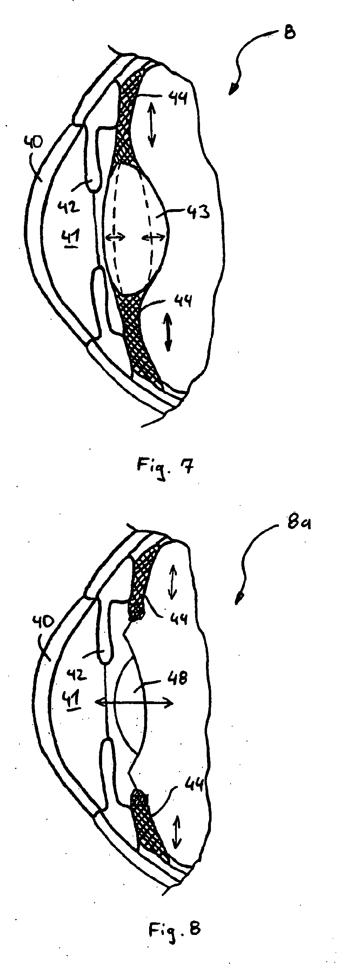

**Fig. 7** und **Fig. 8** zeigen die Möglichkeit zum Einsatz des erfindungsgemäßen Verfahrens bei Erregung des Akkomodationsmuskels 44 zur Verformung der Augenlinse 43 (siehe **Fig. 7**) bzw. zur Verstellung einer künstlichen Intraocularlinse 48, die die natürliche Augenlinse 43 beispielsweise bei einer Katarakterkrankung, ersetzt. Durch Anspannung bzw. Entspannung des Akkomodationsmuskels 44 kann die Augenlinse 43 gestreckt bzw. gestaucht werden, so dass sich die Dicke der Augenlinse 43 und die Wölbung der Vorder- bzw. Hinterseite verändert. Dadurch können unterschiedlich weit entfernte Bildebenen scharf gestellt werden. Um die Erregung des Akkomodationsmuskels 44 zu variieren, kann das Untersuchungsgerät mit einer im Abstand veränderbaren Fixationsmarke ausgerüstet sein.

Bei der in **Fig. 8** dargestellten Intraocularlinse erfolgt die Scharfstellung der Bildebene durch Verschiebung der Intraocularlinse in Richtung der Hauptachse des Auges. Diese transversale Verschiebung der Intraocularinse wird über eine Gelenkaufhängung realisiert, die mit dem Akkomodationsmuskel 44 zusammenwirkt. Durch Anspannen bzw. Entspannen des Akkomodationsmuskels 44 wird somit die gewünschte Verschiebung der Intraocularlinse 48 in Richtung der Augenhauptachse bewirkt.

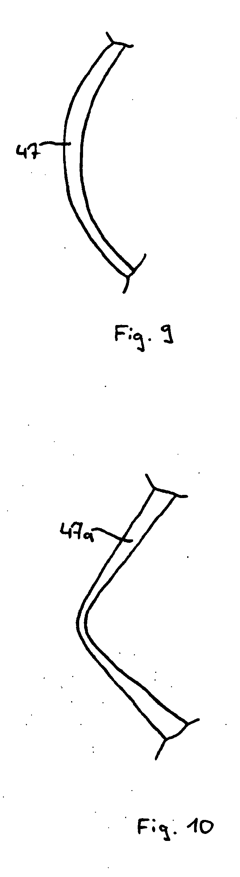

**Fig. 9** zeigt eine gesunde Hornhaut 47 im schematisierten Querschnitt. Die Hornhaut 47 hat einen relativ konstanten Dickenverlauf und wölbt sich somit kreisbogenförmig auf.

Im Unterschied dazu ist in **Fig. 10** eine Hornhaut 47a dargestellt, die eine Keratoconuserkrankung aufweist. Aufgrund dieser Erkrankung ist die Hornhaut 47a in der Mitte stark verdünnt, so dass die Hornhaut 47a sich kegelförmig nach außen wölbt. Durch Bestimmung des Dickenverlaufs der Hornhaut 47a bzw. deren dreidimensionale Geometrie kann die Keratoconuserkrankung durch Anwendung des erfindungsgemäßen Untersuchungsverfahrens bestimmt werden.

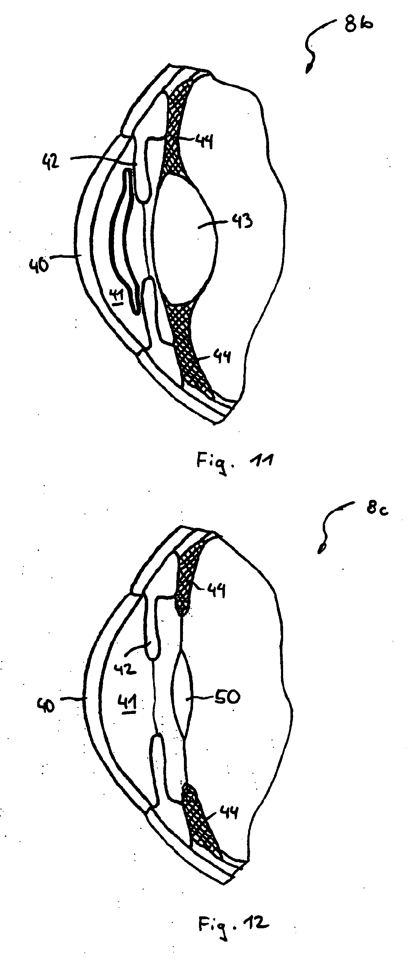

**Fig. 11** zeigt die Anordnung einer phaken Intraocularlinse 49 in der Augenvorkammer 41. Um vor Implantierung der phaken Intraocularlinse 49 bestimmen zu können, ob die Intraocularlinse 49 ausreichend Platz in der Augenvorkammer 41 findet, kann die Augenvorkammer 41 mit dem erfindungsgemäßen Untersuchungsverfahren dreidimensional vermessen werden.

**Fig. 12** zeigt die Anordnung einer zweiten Ausführungsform einer Intraocularlinse 50 im Glaskörper 45 unmittelbar hinter der Iris 42.

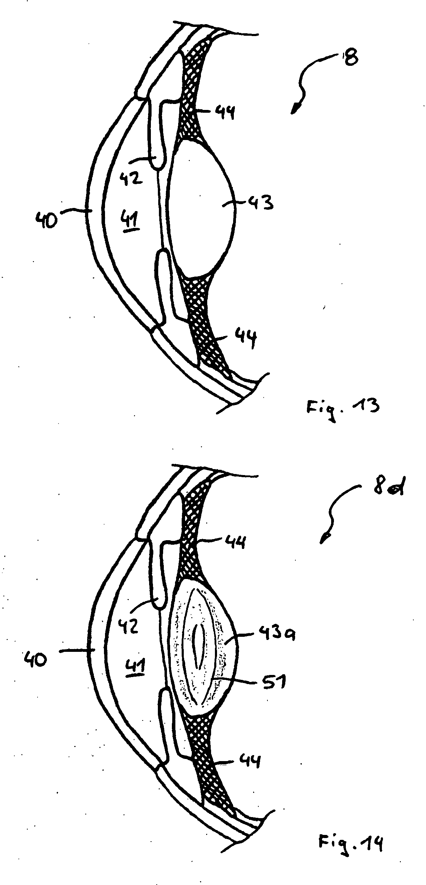

**Fig. 13** stellt die Augenlinse 43 im gesunden Zustand dar. Die Augenlinse 43 ist klar und weist keinerlei Trübungen auf. Im Unterschied dazu ist in **Fig. 14** die Augenlinse 43a mit einer Katarakterkrankung schematisch dargestellt. Die Augenlinse 43a weist aufgrund der Katarakterkrankung Trübungen 51 auf, durch die das Sehvermögen eingeschränkt wird. Diese Trübungen 51 können durch Anwendung des erfindungsgemäßen Untersuchungsverfahrens dreidimensional bestimmt werden.

In **Fig. 15** ist die Wirkungsweise der Rasterblende 26 bei Untersuchung des Auges 8 schematisiert dargestellt. Aufgrund der Aufteilung des Lichtspalts in einzelne Spaltabschnitte wird auf das Auge 8 ein Streulichtmuster projiziert, das in unterschiedlichen Schnittbildebenen aufgenommen wird. Durch Auswertung dieses Streulichtmusters in den unterschiedlichen Schnittbildebenen kann die Achslänge X und der Brechungsindex der Augenlinse 43 bestimmt werden.

## Patentansprüche

1. Verfahren zum Betrieb eines ophthalmologischen Analysesystems mit einer Spaltprojektionseinrichtung (2) zur Spaltbeleuchtung des Auges und einer Scheimpflugaufnahmeeinrichtung (1) zur Aufnahme von digitalisierten Schnittbildern des Auges, wobei Spaltprojektionseinrichtung (2) und Scheimpflugaufnahmeeinrichtung (1) gemeinsam um eine Achse (2), die mit der optischen Achse des Auges im wesentlichen zusammenfällt, rotierend verschwenkbar gelagert sind, und wobei
- in einer ersten Position von Spaltprojektionseinrichtung (2) und Scheimpflugaufnahmeeinrichtung (1) eine Aufnahme eines Schnittbildes des Auges gemacht und als erster Bilddatensatz gespeichert wird,
- Spaltprojektionseinrichtung (2) und Scheimpflugaufnahmeeinrichtung (1) zumindest einmal rotierend verschwenkt und in dieser jeweils nachfolgenden (zweiten, dritten, vierten, ...) Position fixiert werden;
- in der jeweils nachfolgenden (zweiten, dritten, vierten, ...) Position von Spaltprojektionseinrichtung (2) und Scheimpflugaufnahmeeinrichtung (1) eine Aufnahme eines Schnittbildes des Auges gemacht und als nachfolgender (zweiter, dritter, vierter, ...) Bilddatensatz gespeichert wird,
- in einer digitalen Bilddatenanalyseeinrichtung ein mehrdimensionales Beschreibungsmodel zumindest eines Bestandteils des Auges durch digitale Bilddatenanalyse der verschiedenen Bilddatensätze abgeleitet und als Ergebnisdatensatz gespeichert wird,
- der Ergebnisdatensatz in geeigneter Form an einer Datenausgabeeinrichtung ausgegeben wird,
**dadurch gekennzeichnet,**
**dass** während der Aufnahme der nachfolgenden (zweiten, dritten, vierten, ...) Schnittbilder im Bereich zwischen Spaltprojektionseinrichtung (2) und Auge eine Rasterblende (26) angeordnet wird, so dass der Beleuchtungsspalt durch die Rasterblende (26) in mehrere Spaltabschnitte aufgeteilt wird, die unter Bildung eines Streulichtmusters das Auge entlang getrennter Strahlengänge durchlaufen, wobei aus den das Streulichtmuster enthaltenden Bilddatensätzen die Brechungsverhältnisse der Augenlinse, insbesondere der Brechungsindex des die Augenlinse bildenden Gewebes, abgeleitet wird.

2. Verfahren nach Anspruch 1,
**dadurch gekennzeichnet,**
**dass** aus den Bilddatensätzen oder anderen Messdatensätzen, die mit einem weiteren in das ophthalmologischen Analysesystem integrierten Untersuchungsgerät aufgenommen wurden, die Achslänge des Auges zwischen Hornhaut und Netzhaut abgeleitet wird.

3. Verfahren nach Anspruch 2,
**dadurch gekennzeichnet,**
**dass** Spaltprojektionseinrichtung (2) und Scheimpflugaufnahmeeinrichtung (1) bei der Messung der Achslänge des Auges entsprechend der Scheimpflugregel derart ereingerichtet sind, dass das Auge über die volle Tiefe von der Hornhaut bis zur Netzhaut im Tiefenschärfebereich der Scheimpflugaufnahmeeinrichtung (1) auf die Projektionsebene der Scheimpflugaufnahmeeinrichtung (1) abgebildet wird.

4. Verfahren nach einem der Ansprüche 1 bis 3,
**dadurch gekennzeichnet,**
**dass** aus den Bilddatensätzen die Dicke der natürliche Augenlinse des Auges oder die Position einer die natürliche Augenlinse ersetzenden, künstlichen Intraocularlinse im Auge abgeleitet wird.

5. Verfahren nach Anspruch 4,
**dadurch gekennzeichnet,**
**dass** der Akkomodationsmuskel, der zur Verformung der natürlichen Augenlinse oder zur Verstellung einer die natürliche Augenlinse ersetzenden, künstlichen Intraocularlinse vorgesehen ist, während einer Messreihe in unterschiedliche Erregungszustände versetzt wird, so dass die natürliche Augenlinse in unterschiedlichen Verformungszuständen oder die künstlichen Intraocularlinse in unterschiedlichen Positionen, aufgenommen werden kann.

6. Verfahren nach Anspruch 5,
**dadurch gekennzeichnet,**
**dass** im ophthalmologischen Analysesystems eine Fixationsmarke vorhanden ist, die vom Patienten während einer Messreihe fixiert wird, wobei zur variablen Erregung des Akkomodationsmuskels der tatsächliche oder virtuelle Abstand zwischen Auge und Fixationsmarke variiert wird.

7. Verfahren nach Anspruch 6,
**dadurch gekennzeichnet,**
**dass** der virtuelle Abstand zwischen Auge und Fixationsmarke durch Verstellung zumindest einer Linse im Strahlengang zwischen Fixationsmarke und Auge variiert wird.

8. Verfahren nach einem der Ansprüche 1 bis 7,
**dadurch gekennzeichnet,**
**dass** aus den Bilddatensätzen die Geometrie der Augenvorkammer zwischen Hornhaut und Iris abgeleitet wird.

9. Verfahren nach Anspruch 8,
**dadurch gekennzeichnet,**
**dass** ausgehend von Geometrie der Augenvorkammer die Anordnung einer phaken Intraokularlinse in der Augenvorkammer zwischen Hornhaut und Iris im Analysesystem simuliert wird.

10. Verfahren nach einem der Ansprüche 1 bis 9,
**dadurch gekennzeichnet,**
**dass** aus den Bilddatensätzen die Geometrie der Iris und des unmittelbar daran anschließenden Teils des Glaskörpers abgeleitet wird.

11. Verfahren nach Anspruch 10,
**dadurch gekennzeichnet,**
**dass** ausgehend von Geometrie der Iris und des unmittelbar daran anschließenden Teils des Glaskörpers die Anordnung einer Intraokularlinse im Glaskörper unmittelbar hinter der Iris simuliert wird.

12. Verfahren nach einem der Ansprüche 1 bis 11,
**dadurch gekennzeichnet,**
**dass** das Auge bei der Messreihe mit Streulichtmuster auf einen Fixationspunkt in unendlicher Entfernung fixiert wird, wobei aus den das Streulichtmuster enthaltenden Bilddatensätzen die Achslänge des Auges zwischen Hornhaut und Netzhaut abgeleitet wird.

## Claims

1. A method for operating an ophthalmological analysis system having a slit projection device (2) for slit illumination of the eye and a Scheimpflug recording device (1) for recording digitised sectional images of the eye, the slit projection device (2) and the Scheimpflug recording device (1) being mounted such that they may pivot jointly rotating around an axis (9) which is essentially coincident with the optical axis of the eye, and
- in a first position of the slit projection device (2) and the Scheimpflug recording device (1), a recording of a sectional image of the eye being made and stored as a first image data set,
- the slit projection device (2) and the Scheimpflug recording device (1) being pivoted rotating at least once and being fixed in this particular subsequent (second, third, fourth,...) position;
- in the particular subsequent (second, third, fourth,...) position of the slit projection device (2) and the Scheimpflug recording device (1), a recording of a sectional image of the eye being made and stored as a subsequent (second, third, fourth,...) image data set,
- in a digital image data analysis device, a multidimensional description model of at least one component of the eye being derived through digital image data analysis of the different image data sets and stored as a resulting data set,
- the resulting data set being output in suitable form by a data output device,
**characterised in that**
during the recording of the subsequent (second, third, fourth,...) sectional images, a raster aperture (26) is arranged in the region between the slit projection device (2) and the eye, such that the illumination slit is divided by the raster aperture (26) into multiple slit sections, which pass through the eye along separate beam paths to form a scattered light pattern, the ratios of refraction of the eye lens, in particular the index of refraction of the tissue forming the eye lens, being derived from the image data sets containing the scattered light pattern.

2. The method according to claim 1,
**characterised in that**
the axial length of the eye between the cornea and the retina is derived from the image data sets or other measurement data sets which were recorded using a further examination apparatus that is integrated into the ophthalmological analysis system.

3. The method according to claim 2,
**characterised in that**
the slit projection device (2) and the Scheimpflug recording device (1) are set up when measuring the axial length of the eye in accordance with the Scheimpflug rule in such a manner that the eye is imaged onto the projection plane of the Scheimpflug recording device (1) over the complete depth from the cornea up to the retina in the area of depth of field of the Scheimpflug recording device (1).

4. The method according to any one of claims 1 to 3,
**characterised in that**
the thickness of the natural eye lens of the eye or the position of an artificial intraocular lens in the eye that replaces the natural eye lens is derived from the image data sets.

5. The method according to claim 4,
**characterised in that**
the accommodation muscle, which is provided for deforming the natural eye lens or for adjusting an artificial intraocular lens that replaces the natural eye lens, is set in different states of excitation during a series of measurements, such that the natural eye lens may be recorded in different deformation states or the artificial intraocular lens may be recorded in different positions.

6. The method according to claim 5,
**characterised in that**
a fixation mark is provided in the ophthalmological analysis system, which is fixed on by the patient during a series of measurements, the actual or virtual distance between the eye and the fixation mark being varied for variable excitation of the accommodation muscle.

7. The method according to claim 6,
**characterised in that**
the virtual distance between the eye and the fixation mark is varied by adjusting at least one lens in the beam path between the fixation mark and the eye.

8. The method according to any one of claims 1 to 7,
**characterised in that**
the geometry of the anterior chamber of the eyeball between the cornea and the iris is derived from the image data sets.

9. The method according to claim 8,
**characterised in that**
the arrangement of a phakic intraocular lens in the anterior chamber of the eyeball between the cornea and the iris is simulated in the analysis system on the basis of the geometry of the anterior chamber of the eyeball.

10. The method according to any one of claims 1 to 9,
**characterised in that**
the geometry of the iris and of the part of the vitreous body directly adjoining it is derived from the image data sets.

11. The method according to claim 10,
**characterised in that**
the arrangement of an intraocular lens in the vitreous body directly behind the iris is simulated on the basis of the geometry of the iris and of the directly adjoining part of the vitreous body.

12. The method according to any one of claims 1 to 11,
**characterised in that**
during the series of measurements, the eye is fixed on a fixation point at infinite distance using a scattered light pattern, the axial length of the eye between the cornea and the retina being derived from the image data sets containing the scattered light pattern.

## Revendications

1. Procédé d'opération d'un système d'analyse ophthalmologique comprenant un dispositif (2) de projection à fente pour l'éclairage à fente de l'oeil et un dispositif (1) d'enregistrement Scheimpflug pour enregistrer des images en coupe numérisées de l'oeil, le dispositif (2) de projection à fente et le dispositif (1) d'enregistrement Scheimpflug étant montés de manière à pouvoir pivoter conjointement en tournant autour d'un axe (9) qui coïncide essentiellement avec l'axe optique de l'oeil, et
- dans une première position du dispositif (2) de projection à fente et du dispositif (1) d'enregistrement Scheimpflug, un enregistrement d'une image en coupe de l'oeil étant fait et mémorisé comme un premier ensemble de données d'image,
- le dispositif (2) de projection à fente et le dispositif (1) d'enregistrement Scheimpflug étant pivotés en tournant au moins une fois et étant fixés dans cette position respectivement subséquente (deuxième, troisième, quatrième, ...);
- dans la position respectivement subséquente (deuxième, troisième, quatrième, ...) du dispositif (2) de projection à fente et du dispositif (1) d'enregistrement Scheimpflug, un enregistrement d'une image en coupe de l'oeil étant fait et étant mémorisé comme un ensemble de données d'image subséquent (deuxième, troisième, quatrième, ...),
- dans un dispositif d'analyse de données d'image numérique, un modèle de description multidimensionnel d'au moins un composant de l'oeil étant dérivé au moyen d'une analyse de données d'image numérique des différents ensembles de données d'image et étant mémorisé comme un ensemble de données de résultat,
- l'ensemble de données de résultat étant émis sous forme appropriée par un dispositif de sortie de données,
**caractérisé en ce que**,
pendant l'enregistrement des image en coupe subséquentes (deuxièmes, troisièmes, quatrièmes, ...), un diaphragme (26) de trame est arrangée dans la région entre le dispositif (2) de projection à fente et l'oeil, de telle sorte qu'au moyen du diaphragme (26) de trame, la fente d'éclairage est divisée en plusieurs sections de fente, qui passent par l'oeil le long de trajectoires du faisceau séparées en formant un motif de lumière de diffusion, à partir des ensembles de données d'image qui contiennent le motif de lumière de diffusion, les rapports de réfraction du cristallin de l'oeil, en particulier l'indice de réfraction du tissu qui forme le cristallin de l'oeil, étant dérivés.

2. Procédé selon la revendication 1,
**caractérisé en ce qu'**
à partir des ensembles de données d'image ou d'autres ensembles de données de mesure, qui ont été enregistrés à l'aide d'un autre appareil d'examen qui est intégré dans le système d'analyse ophthalmologique, la longueur axiale de l'oeil entre la cornée et la rétine est dérivée.

3. Procédé selon la revendication 2,
**caractérisé en ce que**
le dispositif (2) de projection à fente et le dispositif (1) d'enregistrement Scheimpflug sont aménagés lors de la mesure de la longueur axiale de l'oeil conformément à la règle de Scheimpflug de telle manière que l'oeil est reproduit sur le plan de projection du dispositif (1) d'enregistrement Scheimpflug, par la pleine profondeur de la cornée à la rétine, dans la région de profondeur de champ du dispositif (1) d'enregistrement Scheimpflug.

4. Procédé selon l'une quelconque des revendications 1 à 3,
**caractérisé en ce qu'**
à partir des ensembles de données d'image, l'épaisseur du cristallin naturel de l'oeil ou la position d'une lentille intraoculaire artificielle dans l'oeil, qui remplace le cristallin naturel de l'oeil, est dérivée.

5. Procédé selon la revendication 4,
**caractérisé en ce que**
le muscle d'accommodation, qui est prévu pour déformer le cristallin naturel de l'oeil ou pour régler une lentille intraoculaire artificielle qui remplace le cristallin naturel de l'oeil, est mis dans des différents états d'excitation pendant une série de mesure, de telle sorte que le cristallin naturel de l'oeil peut être enregistré dans des différents états de déformation ou la lentille intraoculaire artificielle peut être enregistrée dans des différentes positions.

6. Procédé selon la revendication 5,
**caractérisé en ce que**,
dans le système d'analyse ophthalmologique, une marque de fixation est prévu, qui est fixé par le patient pendant une série de mesure, pour l'excitation variée du muscle d'accommodation, la distance effective ou virtuelle entre l'oeil et la marque de fixation étant variée.

7. Procédé selon la revendication 6,
**caractérisé en ce que**
la distance virtuelle entre l'oeil et la marque de fixation est variée en réglant au moins une lentille dans la trajectoire du faisceau entre la marque de fixation et l'oeil.

8. Procédé selon l'une quelconque des revendications 1 bis 7,
**caractérisé en ce qu'**
à partir des ensembles de données d'image, la géométrie de la chambre antérieure de l'oeil entre la cornée et l'iris est dérivée.

9. Procédé selon la revendication 8,
**caractérisé en ce que**,
partant de la géométrie de la chambre antérieure de l'oeil, l'arrangement d'une lentille intraoculaire phakique dans la chambre antérieure de l'oeil entre la cornée et l'iris est simulé dans le système d'analyse.

10. Procédé selon l'une quelconque des revendications 1 à 9,
**caractérisé en ce qu'**
à partir des ensembles de données d'image, la géométrie de l'iris et de la partie du corps vitré qui s'y raccorde directement est dérivé.

11. Procédé selon la revendication 10,
**caractérisé en ce que**,
partant de la géométrie de l'iris et de la partie du corps vitré qui s'y raccorde directement, l'arrangement d'une lentille intraoculaire dans le corps vitré directement derrière l'iris est simulé.

12. Procédé selon l'une quelconque des revendications 1 à 11,
**caractérisé en ce que**,
lors de la série de mesure, l'oeil est fixée sur un point de fixation infiniment éloigné à l'aide d'un motif de lumière de diffusion, à partir des ensembles de données d'image, qui contiennent le motif de lumière de diffusion, la longueur axiale de l'oeil entre la cornée et la rétine est dérivée.
